# EUROPEAN PATENT APPLICATION

(11) **EP 4 339 201 A2**
(43) Date of publication of application: **20.03.2024**
(21) Application number: 23194704.5
(22) Date of filing: 31.08.2023
(51) Int. Cl.: C07J 43/00

(54) **DERIVATIZATION METHOD, MASS SPECTROMETRY METHOD, DERIVATIZATION REAGENT, AND DERIVATIZATION REAGENT KIT FOR ESTROGEN**

(30) Priority: 16.09.2022 JP 2022147575
(71) Applicant: Tokyo University of Science Foundation, Tokyo 162-8601 (JP); JEOL Ltd., Akishima-shi, Tokyo 196-8558 (JP)
(72) Inventor: HIGASHI, Tatsuya, Shinjuku-ku, 1628601 (JP); TAKIWAKI, Masaki, Akishima, 1968558 (JP); TAKAHASHI, Koji, Akishima, 1968558 (JP); FUKUZAWA, Seketsu, Akishima, 1968558 (JP)
(74) Representative: Boult Wade Tennant LLP

(57) **Abstract**

The present invention is intended to provide a novel technique for improving the detection sensitivity of estrogen in an analytical method in which estrogen is ionized. The present invention provides an estrogen derivatization method using a quaternary cation-containing 5-fluoro-2,4-dinitrophenyl compound represented by Formula (1): 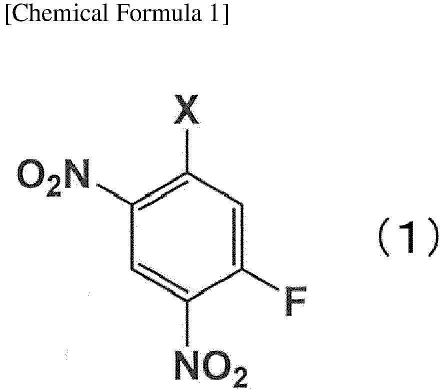 [in Formula (1), X is a quaternary cation]. The present invention also provides a mass spectrometry method comprising derivatizing estrogen by the above estrogen derivatization method.

## Description

### Technical Field

The present invention relates to a derivatization method, a mass spectrometry method, a derivatization reagent, and a derivatization reagent kit for estrogen.

### Background Art

Liquid chromatography-tandem mass spectrometry (LC-MS/MS) and matrix-assisted laser desorption-ionization mass spectrometry (MALDI-MS) are extremely powerful analytical techniques for quantifying trace amounts of metabolites or contents. In these analytical techniques, a target compound is ionized. In the analysis in which a target compound is ionized, some target compounds may be ionized inefficiently and thus cannot be detected, or detection accuracy may be insufficient for quantification in many cases. To address such problems, a target compound may be derivatized to improve the detection sensitivity.

For example, Non-Patent Document 1 discloses a derivatization reagent for hydroxy steroids. Non-Patent Document 1 discloses a technique that can improve the detection sensitivity of estrogen by derivatizing the estrogen under specific conditions as compared to without derivatization.

### Citation List

### Non-Patent Document

[Non-Patent Document 1] Development and application of electrospray-active derivatization reagents for hydroxy steroids, Nishio, T.; Higashi, T.; Funaishi, A.; Tanaka, J.; Shimada, K., J. Pharm. Biomed. Anal., 2007, 44, 786-795.

### Summary of the Invention

### Technical Problem

The present invention is intended to provide a novel technique for improving the detection sensitivity of estrogen in an analytical method in which estrogen is ionized.

### Solution to Problem

The inventors of the present invention have found that a quaternary cation-containing 5-fluoro-2,4-dinitrophenyl compound (X-DNPF) is suitable for derivatization to improve the detection sensitivity of estrogen.

In other words, the present invention provides a derivatization method for estrogen using a quaternary cation-containing 5-fluoro-2,4-dinitrophenyl compound represented by Formula (1): 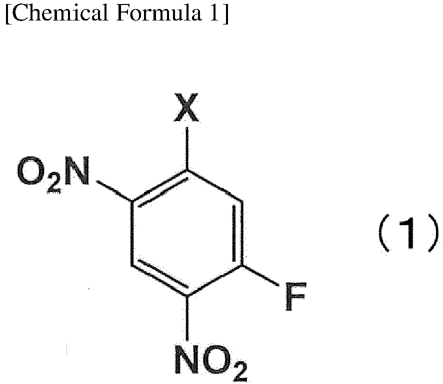 [in Formula (1), X is a quaternary cation].

In Formula (1), the quaternary cation may be a quaternary ammonium cation. In this case, the quaternary ammonium cation may be a group represented by any of Formulae (2) to (4): 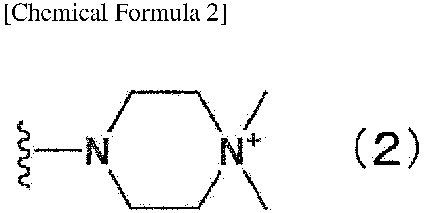 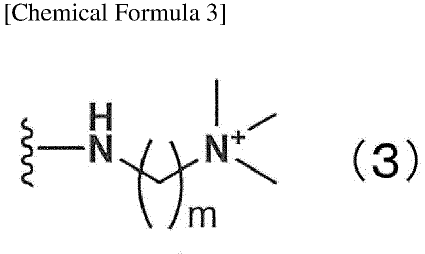 [in Formula (3), m is an integer of 1 to 8] 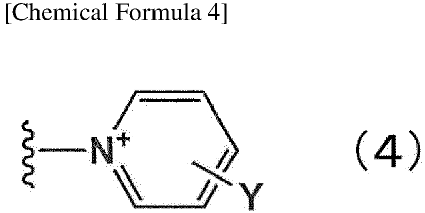 [in Formula (4), Y is a substituted or unsubstituted, linear or branched alkyl group having 1 to 20 carbon atoms].

In Formula (1), the quaternary cation may be a quaternary phosphonium cation. In this case, the quaternary phosphonium cation may be a group represented by Formula (5): 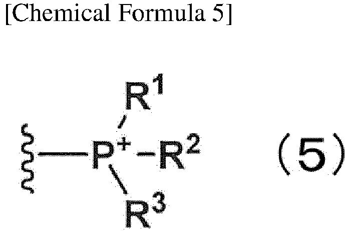 [in Formula (5), R¹, R², and R³ are each independently a group represented by Formula (6): 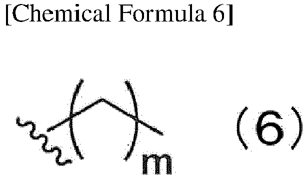 in Formula (6), m is an integer of 0 to 8].

The derivatization method for estrogen may be a method further using an organic base.

The organic base may be a tertiary amine.

The present invention also provides a mass spectrometry method comprising derivatizing estrogen by the above derivatization method for estrogen.

The present invention also provides a derivatization reagent for derivatizing estrogen, and the derivatization reagent comprises a quaternary cation-containing 5-fluoro-2,4-dinitrophenyl compound represented by Formula (1): 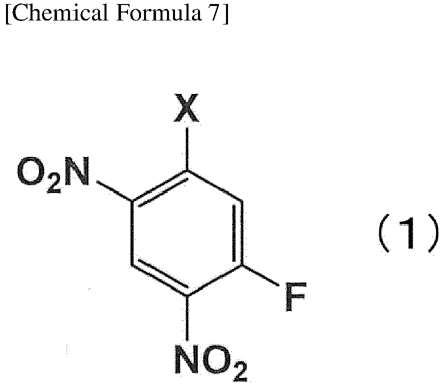 [in Formula (1), X is a quaternary cation].

The present invention also provides a derivatization reagent kit for derivatizing estrogen, and the derivatization reagent kit comprises the above derivatization reagent.

### Advantageous Effects of Invention

According to the present invention, the detection sensitivity of estrogen can be improved in an analytical method in which estrogen is ionized, such as a mass spectrometry method.

The effect of the invention is not limited to that described in this paragraph and may be any of the effect described in the present specification.

### Brief Description of Drawings

[Fig. 1] Fig. 1 shows example reaction schemes of estrogen derivatization.
[Fig. 2] Fig. 2 shows an example reaction scheme of estrogen derivatization.
[Fig. 3] Fig. 3 shows an SRM chromatogram of MP-DNP-E2 prepared by a conventional two-step derivatization method.
[Fig. 4] Fig. 4 shows an SRM chromatogram of MP-DNP-E2 prepared by a one-pot derivatization method according to the present invention.
[Fig. 5] Fig. 5 shows SRM chromatograms of MP-DNP-E2 in an E2 standard and a pooled serum.
[Fig. 6] Fig. 6 shows graphs comparing peak areas in SRM chromatograms of MP-DNP-E2 and Dns-E2 in an E2 standard and a pooled serum.
[Fig. 7] Fig. 7 shows comparative SRM chromatograms of MP-DNP-E2 and Dns-E2 in samples at a low E2 concentration.
[Fig. 8] Fig. 8 shows SRM chromatograms when a sample containing E1, E2, and E3 each at a concentration of 62.5 pg/mL was derivatized with MP-DNPF.
[Fig. 9A] Fig. 9A shows a graph in which response ratios between E1 component and E1 internal standard and sample concentrations are plotted.
[Fig. 9B] Fig. 9B shows a graph in which response ratios between E2 component and E2 internal standard and sample concentrations are plotted.
[Fig. 9C] Fig. 9C shows a graph in which response ratios between E3 component and E3 internal standard and sample concentrations are plotted.
[Fig. 10] Fig. 10 shows SRM chromatograms when a pooled serum was derivatized with MP-DNPF.
[Fig. 11] Fig. 11 shows SRM chromatograms when a serum containing standard samples was derivatized with MP-DNPF.
[Fig. 12] Fig. 12 shows SRM chromatograms when a pooled serum was derivatized with MP-DNPF.
[Fig. 13] Fig. 13 shows an NMR spectrum.
[Fig. 14] Fig. 14 shows an NMR spectrum.
[Fig. 15] Fig. 15 shows an NMR spectrum.

### Description of Embodiments

Preferred embodiments of the present invention will be described below. However, the present invention is not limited to the following preferred embodiments, which can be arbitrarily changed within the scope of the invention.

The present invention will be described in the following order.
1. Description of the present invention
2. First embodiment (estrogen derivatization method)
3. Second embodiment (mass spectrometry method)
4. Third embodiment (derivatization reagent)
5. Fourth embodiment (derivatization reagent kit)
6. Examples

### 1. Description of the present invention

The above-described mass spectrometry such as LC-MS/MS and MALDI-MS is an extremely powerful analytical technique for quantifying trace amounts of metabolites or contents. Some compounds are, however, ionized inefficiently and thus cannot be detected or can be detected only at insufficient accuracy for quantification.

For samples such as foods and supplements that are available in a certain amount, a larger amount of a sample can be used to compensate insufficient sensitivity. Even such a sample is desirably used in a smaller amount to detect or quantify a target compound.

In the analysis of a target compound such as a human metabolite, the amount of a sample containing the target compound cannot be easily increased in many cases. For such a target compound, it is very important to improve the ionization efficiency, thereby improving the detection sensitivity.

To improve the detection sensitivity, not only apparatus improvement such as higher sensitivity of a mass spectrometer but also modification (derivatization) of a target compound for higher ionization efficiency have been widely developed. A typical biometabolite is an organic compound mainly comprising carbon, hydrogen, oxygen, and nitrogen, and an oxygen atom or a nitrogen atom, which has a lone electron pair, is likely to be added with a hydrogen ion (proton) and has high ionization efficiency. In particular, a nitrogen atom has higher protonation properties than an oxygen atom, and a compound containing a nitrogen atom is more likely to be ionized than compounds containing no nitrogen atom.

Estrogen known as a female hormone contains no nitrogen atom, and thus a derivatization technique with a nitrogen atom-containing derivatization reagent to introduce a nitrogen atom has been developed to improve the detection sensitivity of estrogen. For example, a 5-fluoro-2,4-dinitrophenyl compound having any substituent at position 1 has high electrophilicity at position 5 due to very strong electron-withdrawing effect by two nitro groups at positions 2 and 4 on the benzene ring. By using the properties, a derivatization reagent having a nitrogen atom-containing substituent at position 1 has been developed (see Non-Patent Document 1).

In the technique disclosed in Non-Patent Document 1, 1-(5-fluoro-2,4-dinitrophenyl)-4-methylpiperazine (P-DNPF) that has methylpiperazine as a tertiary amine at position 1 is reacted with estrogen in the presence of sodium hydrogen carbonate, and then the product is derivatized with methyl iodide (MeI) (MP-DNP-estrogen). This improves the detection sensitivity by a factor of about 2,000 as compared to without derivatization.

In contrast, the inventors of the present invention have found that using a quaternary cation-containing 5-fluoro-2,4-dinitrophenyl compound (X-DNPF) can improve the detection sensitivity of estrogen in an analytical method in which estrogen is ionized. In other words, the present invention provides an estrogen derivatization method using a quaternary cation-containing 5-fluoro-2,4-dinitrophenyl compound.

The estrogen derivatization method according to the present invention enables one-step derivatization that have been carried out in two steps. Specifically, in the derivatization method disclosed in Non-Patent Document 1, derivatization with P-DNPF is followed by quaternization with methyl iodide, that is, estrogen is derivatized through a two-step reaction. In contrast, the estrogen derivatization method according to the present invention uses a quaternary cation-containing 5-fluoro-2,4-dinitrophenyl compound and thus enables estrogen derivatization through a one-step reaction. The estrogen derivatization method according to the present invention therefore can reduce the reaction steps necessary for the derivatization and can simplify the reaction procedure.

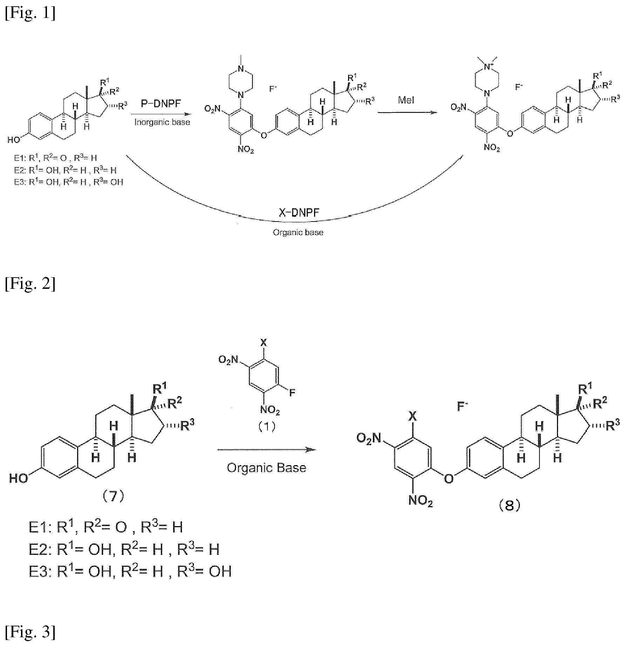

Fig. 1 shows example reaction schemes of derivatizing estrogen in Non-Patent Document 1 and in the present invention. As shown in Fig. 1, the derivatization method in Non-Patent Document 1 yields an estrogen derivative through a two-step reaction including reaction with P-DNPF and an inorganic base and reaction with methyl iodide. In contrast, the present invention uses a quaternary cation-containing 5-fluoro-2,4-dinitrophenyl compound (represented as X-DNPF in Fig. 1) and can yield the same estrogen derivative as in Non-Patent Document 1 through a one-step reaction.

The estrogen derivatization method according to the present invention eliminates the use of an inorganic base (sodium hydrogen carbonate) for the derivatization with P-DNPF in the derivatization method of Non-Patent Document 1, and this probably improves the detection sensitivity and simplifies the reaction procedure. In the estrogen derivatization method according to the present invention, estrogen can be derivatized, for example, with an organic base in place of the inorganic base. Using no inorganic base suppresses contamination of the ion source in an analyzer in which ionization is carried out, and this can improve the detection sensitivity as compared to when the inorganic base is used. The inventors of the present invention have thought that the reason why estrogen is not derivatized through a one-step reaction in the derivatization method of Non-Patent Document 1 is the use of an inorganic base. In the estrogen derivatization method according to the present invention, unnecessary use of the inorganic base is thought to achieve a one-step reaction, that is, to simplify the reaction procedure.

As described above, the present invention enables derivatization of estrogen through a one-step reaction and enables an improvement of the estrogen detection sensitivity in an analyzer in which ionization is carried out.

According to the present invention, for example, in the clinical examination field, a trace metabolite can be detected by mass spectrometry at high sensitivity and can be quantified at high sensitivity. The present invention is applicable to any type of mass spectrometer. For example, to widely spread the quantification of a trace metabolite by mass spectrometry in the clinical examination field, it is desirable to yield the same value regardless of the time, the place, and the apparatus from any manufacturer. The improvement in detection sensitivity by the present invention compensates the difference between apparatuses from various manufacturers and thus can help the spread of quantification of a trace metabolite by mass spectrometry.

The present invention will next be described in further detail.

### 2. First embodiment (estrogen derivatization method)

The present invention provides an estrogen derivatization method using a quaternary cation-containing 5-fluoro-2,4-dinitrophenyl compound represented by Formula (1). In other words, the estrogen derivatization method comprises a step of derivatizing estrogen with a quaternary cation-containing 5-fluoro-2,4-dinitrophenyl compound represented by Formula (1). By the estrogen derivatization method according to the present invention, an estrogen derivative detectable at high sensitivity can be prepared through a one-step reaction.

### 2-1. Quaternary cation-containing 5-fluoro-2,4-dinitrophenyl compound of Formula (1)

The quaternary cation-containing 5-fluoro-2,4-dinitrophenyl compound used in the estrogen derivatization method is represented by Formula (1). 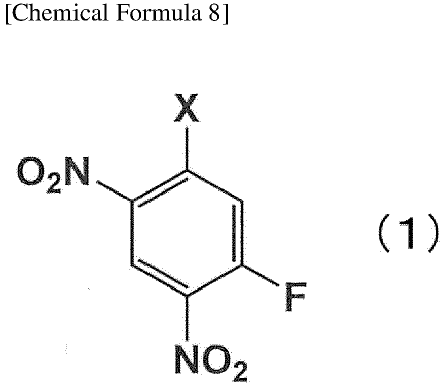 [In Formula (1), X is a quaternary cation]

In one embodiment, the quaternary cation may be a quaternary ammonium cation. The quaternary ammonium cation may be a group represented by any of Formulae (2) to (4). From the viewpoint of further improving the estrogen detection sensitivity in an analytical method in which estrogen is ionized, the quaternary cation is preferably the group represented by Formula (2). 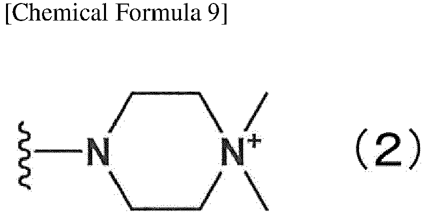 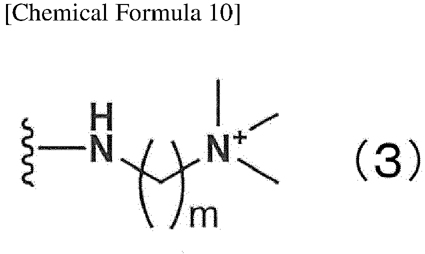 [In Formula (3), m is an integer of 1 to 8] 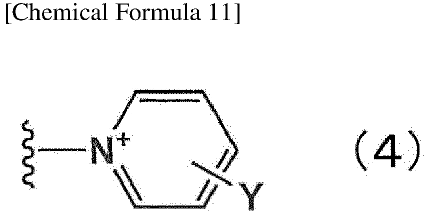 [In Formula (4), Y is a substituted or unsubstituted, linear or branched alkyl group having 1 to 20 carbon atoms]

In Y in Formula (4), the carbon number of the linear or branched alkyl group may, for example, be 1 to 18, 1 to 16, 1 to 14, 1 to 12, or 1 to 10. The carbon number excludes the number of carbon atoms of substituents.

In an example, the linear or branched alkyl group of Y in Formula (4) may be an unsubstituted alkyl group, that is, may be an alkyl group in which only hydrogen atoms are bonded to the carbon chain. In another example, the linear or branched alkyl group of Y in Formula (4) may be a substituted alkyl group, that is, one or more substituents may be bonded to the carbon chain in addition to hydrogen atoms. Examples of the substituent include, but are not limited to, an alkoxy group, an aryl group, a hydroxy group, and a halogen atom. When Y has two or more substituents, the substituents may be the same or different.

In another embodiment, the quaternary cation may be a quaternary phosphonium cation. The quaternary phosphonium cation may be a group represented by Formula (5). 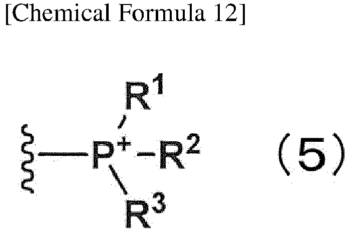 [In Formula (5), R¹, R², and R³ are each independently a group represented by Formula (6): 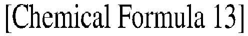 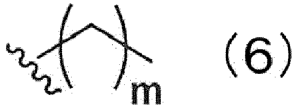 in Formula (6), m is an integer of 0 to 8]

In Formula (6), m may, for example, be 0 to 6, 0 to 4, 0 to 2, or 1.

### 2-2. Organic base

The estrogen derivatization method may further use an organic base. In other words, the estrogen derivatization method may comprise derivatizing estrogen by using a quaternary cation-containing 5-fluoro-2,4-dinitrophenyl compound of Formula (1) and an organic base. The organic base functions as a catalyst in the estrogen derivatization reaction with the quaternary cation-containing 5-fluoro-2,4-dinitrophenyl compound of Formula (1).

Examples of the organic base include a tertiary amine. Examples of the tertiary amine include 4-dimethylaminopyridine and quinuclidine.

### 2-3. Estrogen

The compound to be derivatized with the quaternary cation-containing 5-fluoro-2,4-dinitrophenyl compound of Formula (1) is estrogen. The estrogen is a female hormone and is also referred to as an estrogenic hormone. Generally, the estrogen includes estrone (E1), estradiol (E2), estriol (E3), and estetrol (E4).

The estrogen as the compound to be derivatized may be at least one, two, or three compounds selected from estrone, estradiol, estriol, and estetrol or may be these four compounds. Alternatively, the estrogen as the compound to be derivatized may be at least one or two compounds selected from estrone, estradiol, and estriol or may be these three compounds.

The estrogen may be contained in a biological sample, for example. The biological sample capable of containing estrogen that is to be derivatized according to the invention may, for example, be a body fluid or a sample derived from a body fluid and more specifically be any of serum, plasma, blood, urine, spinal fluid, and saliva. Such a biological sample may be subjected to a certain pretreatment suitable for an analytical method as needed, and then estrogen may be derivatized according to the invention.

### 2-4. Derivatization of estrogen

In the estrogen derivatization method, estrogen derivatization may be carried out by reaction of estrogen with a quaternary cation-containing 5-fluoro-2,4-dinitrophenyl compound of Formula (1) and with the above organic base as needed.

An example reaction scheme of estrogen derivatization with a quaternary cation-containing 5-fluoro-2,4-dinitrophenyl compound of Formula (1) is shown in Fig. 2. In the figure, Formula (7) represents estrogen and specifically represents estrone (E1), estradiol (E2), and estriol (E3). Formula (8) represents an estrogen derivative. As shown in the figure, estrogen (estrone, estradiol, and estriol) is derivatized with the compound of Formula (1).

### 3. Second embodiment (mass spectrometry method)

The present invention also provides a mass spectrometry method comprising derivatizing estrogen by the above-described estrogen derivatization method. In other words, the mass spectrometry method comprises a derivatization step of derivatizing estrogen by the estrogen derivatization method. The estrogen derivatization method is specifically as described in the above section 2, and the description also applies to the present embodiment. Derivatizing estrogen enables an improvement of the estrogen detection sensitivity in mass spectrometry.

The mass spectrometry method comprising carrying out mass spectrometry after the derivatization. Specifically, the mass spectrometry method comprises, after the derivatization step, a mass spectrometry step comprising ionizing the derivatized estrogen.

The ionization may be carried out, for example, by electrospray ionization (ESI), atmospheric pressure chemical ionization (APCI), or matrix-assisted laser desorption ionization (MALDI) for more efficient ionization and may be carried out by any ionization other than these ionizations.

By ionizing the derivatized estrogen by such an ionization method, estrogen can be quantified, and the estrogen distribution (imaging) in a tissue or in cells can be more accurately measured or observed at higher resolution.

The mass spectrometry step may be carried out, for example, by using a liquid chromatography tandem mass spectrometer (LC-MS/MS) or a matrix-assisted laser desorption-ionization mass spectrometer (MALDI-MS), but the apparatus for carrying out the mass spectrometry step is not limited to them. A specific procedure in the mass spectrometry step may be appropriately designed by a person skilled in the art according to, for example, the ionization method to be used or the apparatus to be used.

### 4. Third embodiment (derivatization reagent)

The present invention also provides a derivatization reagent for derivatizing estrogen, and the derivatization reagent comprises the quaternary cation-containing 5-fluoro-2,4-dinitrophenyl compound represented by Formula (1) described above. The quaternary cation-containing 5-fluoro-2,4-dinitrophenyl compound represented by Formula (1) and estrogen are specifically as described in the above section 2, and the description also applies to the present embodiment.

The derivatization reagent may comprise a solvent in addition to the quaternary cation-containing 5-fluoro-2,4-dinitrophenyl compound represented by Formula (1). In other words, in the derivatization reagent, the quaternary cation-containing 5-fluoro-2,4-dinitrophenyl compound represented by Formula (1) may be in a solvent. The solvent may be an organic solvent and may, for example, be acetonitrile, acetone, tetrahydrofuran, dichloromethane, or dimethoxyethane.

The derivatization reagent may, for example, be used in the estrogen derivatization method described in the above section 2 or may be used in the mass spectrometry method described in the above section 3.

### 5. Fourth embodiment (derivatization reagent kit)

The present invention also provides a derivatization reagent kit for derivatizing estrogen, and the derivatization reagent kit comprises the above-described derivatization reagent. The derivatization reagent is specifically as described in the above section 4, and the description also applies to the present embodiment.

The derivatization reagent kit may further comprise an organic base. Examples of the organic base include a tertiary amine. Examples of the tertiary amine include 4-dimethylaminopyridine (DMAP) and quinuclidine.

The derivatization reagent kit may, for example, be used in the estrogen derivatization method described in the above section 2 or may be used in the mass spectrometry method described in the above section 3.

When the derivatization reagent kit is used in the mass spectrometry method, it may further comprise additional reagents usable in the estrogen mass spectrometry. The additional reagent may, for example, include at least one of a calibration solution, an internal standard, and a positive control (such as a control serum).

In the derivatization reagent kit, the quaternary cation-containing 5-fluoro-2,4-dinitrophenyl compound represented by Formula (1), the organic base, and the additional reagent may be in any form such as the solid form and the liquid form. For example, one or more reagents contained in the derivatization reagent kit may be dissolved in any solvent. Alternatively, the derivatization reagent kit may comprise reagents and a solvent for dissolving the reagents. In this case, the reagents may be dissolved as needed before use.

### Examples

### 6. Examples

The present invention will next be described in more detail with reference to examples, but the present invention is not limited to these examples.

### 6-1. Synthesis example

First, 1-(5-fluoro-2,4-dinitrophenyl)-4-dimethylpiperazinium iodide (hereinafter referred to as "MP-DNPF") used in the following examples was synthesized. The synthesis was carried out in accordance with a known literature (Development and application of electrospray-active derivatization reagents for hydroxy steroids, T. Nishio et al., Journal of Pharmaceutical and Biomedical Analysis, 44 (2007) 786-795). The synthesized MP-DNPF was a compound represented by Formula (1), and X in Formula (1) was the group represented by Formula (2).

### 6-2. Example 1

As described above with reference to Fig. 1, estrogen is derivatized through a two-step reaction in the related art, but the estrogen derivatization method according to the present invention derivatizes estrogen through a one-pot reaction. Tests were carried out to compare the sensitivity between the conventional two-step derivatization and the one-pot derivatization according to the present invention.

### [I. Conventional two-step derivatization (derivatization with P-DNPF and quaternization)]

1. Derivatization with P-DNPF
   1) To a solution of estradiol (E2) (400 fg) in acetonitrile (20 µL), a solution of 1-(5-fluoro-2,4-dinitrophenyl)-4-methylpiperazine (P-DNPF) in acetonitrile (2 mg/mL, 20 µL) was added.
   2) An aqueous sodium hydrogen carbonate (1 M, 20 µL) was further added.
   3) The reaction mixture was allowed to stand at 60°C (60 minutes).
2. Desalination with solid-phase extraction cartridge
   1) A Strata^{™}-X cartridge (60 mg) was conditioned with ethyl acetate (2 mL), methanol (2 mL), and water (2 mL) sequentially.
   2) The above derivatization reaction mixture was diluted with methanol/water (50:50, v/v, 500 µL) and was loaded on the cartridge.
   3) The cartridge was washed with water (2 mL).
   4) P-DNP-E2 was eluted with ethyl acetate (1 mL).
   5) The solvent was removed by a stream of nitrogen to give P-DNP-E2.
3. Quaternization with methyl iodide
   1) To P-DNP-E2, iodomethane (100 µL) was added.
   2) The reaction mixture was allowed to stand at 60°C (30 minutes).
   3) Excess iodomethane was removed by a stream of nitrogen to give MP-DNP-E2.
   4) The product was dissolved in a mobile phase (40 µL) to prepare a sample for LC-MS/MS analysis.
   5) Of the sample, 10 µL was injected to LC-MS/MS.

### [II. One-pot derivatization according to the present invention]

1) To a solution of E2 (200 fg) in acetonitrile (20 µL), a solution of MP-DNPF in acetonitrile (2 mg/mL, 20 µL) was added.
2) A solution of dimethylaminopyridine (DMAP) in acetonitrile (1 mg/mL, 20 µL) was further added.
3) The reaction mixture was allowed to stand at 60°C (15 minutes).
4) The solvent was removed by a stream of nitrogen to give MP-DNP-E2.
5) The product was dissolved in a mobile phase (40 µL) to prepare a sample for LC-MS/MS analysis.
6) Of the sample, 10 µL was injected to LC-MS/MS.

### [III. LC-MS/MS analysis conditions]

### <LC analysis conditions>

Apparatus: Shimadzu LC-30AD
Column: YMC-UltraHT Pro C18 (2.0 µm, 100 × 2.0 mm i.d.)
Mobile phase: methanol-10 mM ammonium formate-formic acid (60:40:0.1, v/v/v)
Flow rate: 0.3 mL/min

### <MS/MS analysis conditions>

Apparatus: Shimadzu LCMS-8030 plus
Ionization method: positive ion detection ESI
Collision activation energy: -43 eV
SRM: m/z 551.2 → 504.3

An SRM chromatogram of the MP-DNP-E2 prepared by the conventional two-step derivatization described in the above section I (100 fg in terms of E2 was injected (detection limit, S/N = 3)) is shown in Fig. 3.

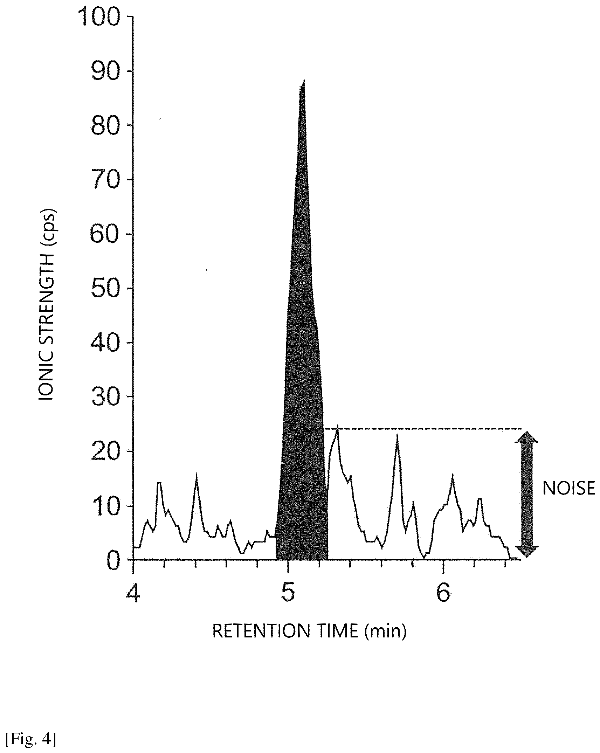

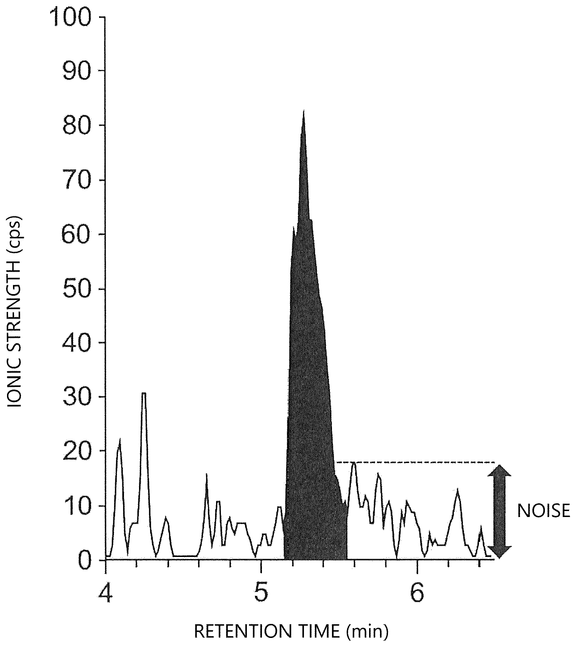

An SRM chromatogram of the MP-DNP-E2 prepared by the one-pot derivatization described in the above section II (50 fg in terms of E2 was injected (detection limit, S/N = 3)) is shown in Fig. 4.

As shown in Figs. 3 and 4, the noises when E2 was derivatized according to the present invention were about half those when E2 was derivatized by the conventional two-step method. The result reveals that the present invention can improve the E2 detection sensitivity by a factor of about 2.

### 6-3. Example 2

MP-DNPF was used to carry out a test to detect E2 in an authentic E2 or in a pooled serum.

### [I. Derivatization with MP-DNPF]

### <Sample pretreatment>

Samples were pretreated in the following procedure.
1. SLE (solid-liquid) extraction
   1) First, 100 µL of serum and 100 µL of water were mixed.
   2) On an SLE column, 200 µL of the diluted serum was loaded.
   3) Next, 600 µL of hexane/ethyl acetate (50/50, v/v) was added, and the sample was eluted. The procedure was repeated three times.
2. Dryness: a stream of nitrogen or centrifugal evaporation for about 20 minutes
3. Derivatization
   1) MP-DNPF was dissolved in acetonitrile so as to give a 2 mg/mL solution.
   2) To the residue, 20 µL of DMAP (1 mg/mL, an acetonitrile solution), 10 µL of the MP-DNPF solution, and 20 µL of acetonitrile were added.
   3) The reaction mixture was allowed to stand at 60°C (15 minutes).
   4) Dryness: a stream of nitrogen or centrifugal evaporation for about 10 minutes
   5) To the residue, 100 µL of 50% (v/v) aqueous acetonitrile was added to prepare a sample for LC-MS/MS analysis.

### <LC analysis conditions>

LC analysis conditions were as follows:
Apparatus: Waters, ACQUITY UPLC I-Class
Analytical column: Waters, ACQUITY UPLC BEH C18 1.7 mm I.D. × 50 mm
Elution conditions: a flow rate of 0.3 mL/min; solvent A: 0.1% formic acid-water;
solvent B: 0.1% formic acid-acetonitrile

Detailed elution conditions were as shown in Table 1.

### [Table 1]

**Table 1. Elution conditions**

| | | | | | | | | |
|---|---|---|---|---|---|---|---|---|
| Time (min) | 0 | 1 | 1.01 | 5 | 5.01 | 6 | 6.01 | 7 |
| B (%) | 20 | 20 | 50 | 50 | 95 | 95 | 20 | 20 |

### <MS/MS analysis conditions>

MS/MS analysis conditions were as follows:
Apparatus: Waters, Xevo TQ-XS triple-quadrupole mass spectrometer
Ionization conditions: ESI positive ion mode

SRM parameters were as shown in Table 2.

### [Table 2]

**Table 2. SRM parameters**

| | SRM (*m*/*z*) | CE |
|---|---|---|
| MP-DNP-E2 | 551.4 > 504.2 | 42 |

### [II. Derivatization with DnsCl]

### <Sample pretreatment>

Samples were pretreated in the following procedure.
1. SLE (solid-liquid) extraction
   1) First, 100 µL of serum and 100 µL of water were mixed.
   2) On an SLE column, 200 µL of the diluted serum was loaded.
   3) Next, 600 µL of hexane/ethyl acetate (50/50, v/v) was added, and the sample was eluted. The procedure was repeated three times.
2. Dryness: a stream of nitrogen or centrifugal evaporation for about 20 minutes
3. Derivatization
   1) Dansyl chloride (DnsCl), a derivatization reagent, was dissolved in acetonitrile so as to give a 1 mg/mL solution.
   2) To the residue, an aqueous sodium hydrogen carbonate (0.1 M, 50 µL) and 50 µL of the DnsCl solution were added.
   3) The reaction mixture was allowed to stand at 60°C (10 minutes).
   4) A sample for LC-MS/MS analysis was prepared.

### <LC analysis conditions>

LC analysis conditions were as follows:
Apparatus: Waters, ACQUITY UPLC I-Class
Analytical column: Waters, ACQUITY UPLC BEH C18 1.7 mm I.D. × 50 mm
Elution conditions: a flow rate of 0.3 mL/min; solvent A: 0.1% formic acid-water;
solvent B: 0.1% formic acid-acetonitrile

Detailed elution conditions were as shown in Table 3.

### [Table 3]

**Table 3. Elution conditions**

| | | | | | | |
|---|---|---|---|---|---|---|
| Time (min) | 0 | 1 | 5 | 6 | 6.01 | 7 |
| B (%) | 50 | 50 | 80 | 95 | 50 | 50 |

### <MS/MS analysis conditions>

MS/MS analysis conditions were as follows:
Apparatus: Waters, Xevo TQ-XS triple-quadrupole mass spectrometer
Ionization conditions: ESI positive ion mode

SRM parameters were as shown in Table 4.

### [Table 4]

**Table 4. SRM parameters**

| | SRM (*m*/*z*) | CE |
|---|---|---|
| Dns-E2 | 506.1 > 171.2 | 40 |

### <Analytical results>

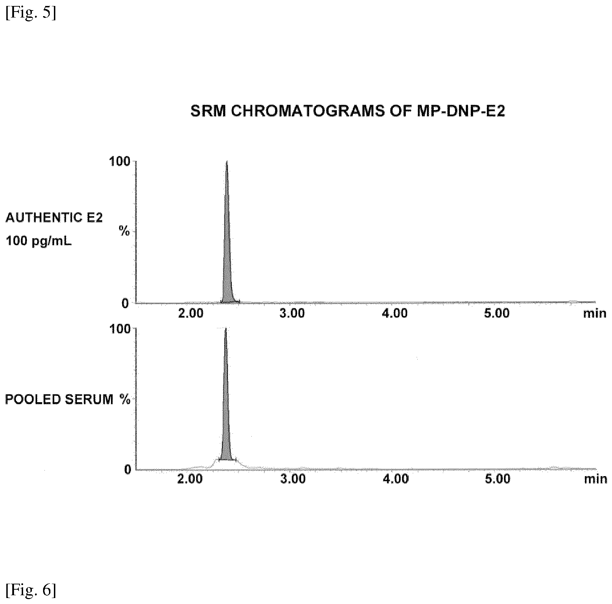

Fig. 5 shows SRM chromatograms of MP-DNP-E2 that was prepared by derivatizing an authentic E2 (100 pg/mL) or a pooled serum with MP-DNPF.

Fig. 6 shows graphs comparing peak areas in SRM chromatograms of MP-DNP-E2 and Dns-E2 that were each prepared by derivatizing the authentic E2 (100 pg/mL) or the pooled serum with MP-DNPF or DnsCl.

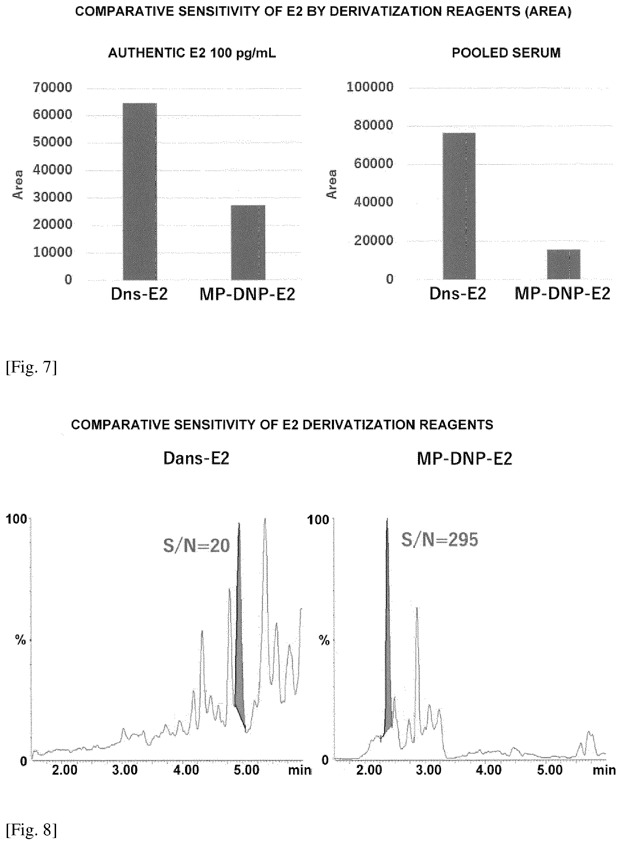

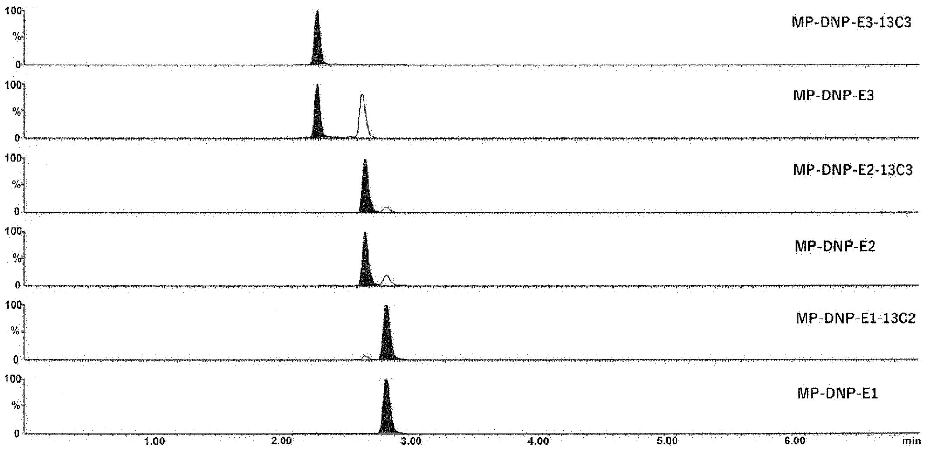

Fig. 7 shows comparative SRM chromatograms of MP-DNP-E2 and Dns-E2 that were prepared as follows: a pooled serum was diluted 10-fold with an E2-free serum (MSG3000, Golden West Diagnostic); and then the prepared sample containing E2 at a low concentration was derivatized with MP-DNPF or DnsCl.

In the result shown in Fig. 6, the sample derivatized with MP-DNPF gave a smaller peak area than the sample derivatized with DnsCl as a conventional reagent. The background noises were, however, smaller in the sample derivatized with MP-DNPF. Derivatization of E2 according to the present invention can reduce the background noises as compared to when DnsCl is used.

As shown in Fig. 7, when a sample was prepared to contain E2 at a low concentration, the S/N in the SRM chromatogram of the sample derivatized with MP-DNP was about 10 times higher than that of the sample derivatized with Dns. The result reveals that the present invention provides good detection sensitivity at a low E2 concentration.

The derivatization of a pooled serum with MP-DNP can greatly reduce the background noise level in an SRM chromatogram as compared to the derivatization with Dns and thus enables sensitive analysis in a low E2 concentration region.

### 6-4. Example 3

MP-DNPF was used to detect and quantify estrone (E1), estradiol (E2), and estriol (E3) standards.

### [Derivatization with MP-DNPF]

### <Sample pretreatment>

Samples were pretreated in the following procedure.
1. Sample preparation
   1) A measurement sample was prepared in 50% (v/v) aqueous acetonitrile such that each concentration of E1, E2, and E3 was 1,000 pg/mL.
   2) The prepared measurement sample was further diluted with 50% (v/v) aqueous acetonitrile to prepare samples at 6.25 pg/mL, 12.5 pg/mL, 25 pg/mL, 62.5 pg/mL, 125 pg/mL, 250 pg/mL, and 500 pg/mL.
   3) With 100 µL of the sample prepared in the above sections 1) and 2), 100 µL of water was mixed, and then, as an internal standard, 20 µL of an E1-¹³C₃, E2-¹³C₃, and E3-¹³C₃ mixed solution (each concentration was 1 ng/mL) prepared in 50% (v/v) aqueous acetonitrile was added. The whole was thoroughly stirred.
2. Dryness: a stream of nitrogen for about 20 minutes
3. Derivatization
   1) MP-DNPF was dissolved in acetonitrile so as to give a 2 mg/mL solution.
   2) To the residue, 20 µL of DMAP (1 mg/mL, an acetonitrile solution), 10 µL of the MP-DNPF solution, and 20 µL of acetonitrile were added.
   3) The reaction mixture was allowed to stand at 60°C (15 minutes).
   4) Dryness: a stream of nitrogen for about 10 minutes
   5) To the residue, 100 µL of 50% (v/v) aqueous acetonitrile was added to prepare a sample for LC-MS/MS analysis.

### <LC analysis conditions>

LC analysis conditions were as follows:
Apparatus: Waters, ACQUITY UPLC I-Class
Analytical column: Waters, ACQUITY UPLC BEH C18 1.7 mm I.D. × 50 mm
Elution conditions: a flow rate of 0.3 mL/min; solvent A: 0.1% formic acid-water;
solvent B: 0.1% formic acid-acetonitrile

Detailed elution conditions were as shown in Table 5.

### [Table 5]

**Table 5. Elution conditions**

| | | | | | | | | |
|---|---|---|---|---|---|---|---|---|
| Time (min) | 0 | 1 | 1.01 | 5 | 5.01 | 6 | 6.01 | 7 |
| B (%) | 20 | 20 | 50 | 50 | 95 | 95 | 20 | 20 |

### <MS/MS analysis conditions>

MS/MS analysis conditions were as follows:
Apparatus: Waters, Xevo TQ-XS triple-quadrupole mass spectrometer
Ionization conditions: ESI positive ion mode

SRM parameters were as shown in Table 6.

### [Table 6]

**Table 6. SRM parameters**

| | SRM (*m*/*z*) | CE |
|---|---|---|
| MP-DNP-E1 | 549.3 > 502.4 | 42 |
| MP-DNP-E1-¹³C₃ | 552.3 > 505.4 | 42 |
| MP-DNP-E2 | 551.4 > 504.2 | 42 |
| MP-DNP-E2-¹³C₃ | 554.3 > 507.2 | 42 |
| MP-DNP-E3 | 567.4 > 520.3 | 42 |
| MP-DNP-E3-¹³C₃ | 570.4 > 523.3 | 42 |

### <Analytical results>

Fig. 8 shows SRM chromatograms when a sample containing E1, E2, and E3 each at a concentration of 62.5 pg/mL was derivatized with MP-DNPF.

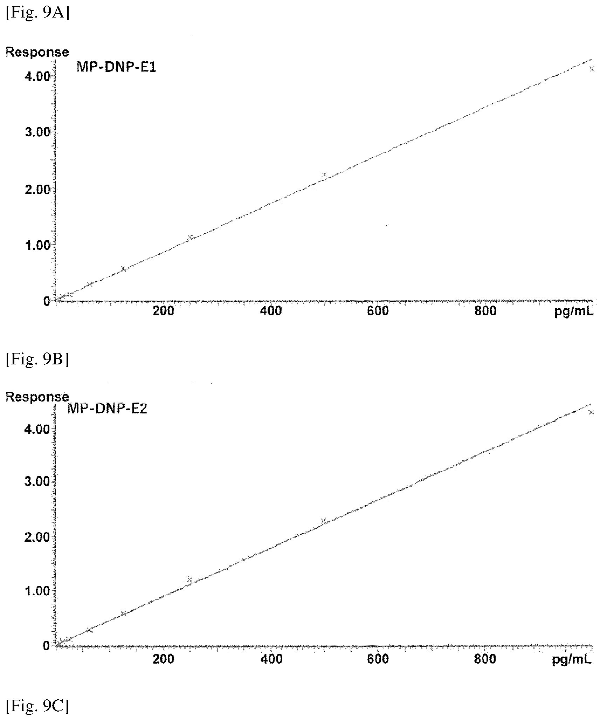

Figs. 9A to C are graphs in which response ratios between each component of E1, E2, and E3 and the corresponding internal standard and sample concentrations were plotted.

The analytical results reveal that each component of E1, E2, and E3 can be derivatized with MP-DNPF, and the components can be simultaneously detected in a concentration-dependent manner. This reveals that the present invention enables derivatization of E1, E2, and E3 and simultaneous detection of E1, E2, and E3 in a concentration-dependent manner.

### 6-5. Example 4

Tests were carried out to detect E1, E2, and E3 in a pooled serum and a serum containing standard samples.

### [Derivatization with MP-DNPF]

### <Sample pretreatment>

Samples were pretreated in the following procedure.
1. SLE (solid-liquid) extraction
   1) To 100 µL of serum, 100 µL of water and, as an internal standard, 30 µL of an E1-¹³C₃, E2-¹³C₃, and E3-¹³C₃ mixed solution (each concentration was 1 ng/mL) prepared in 50% (v/v) aqueous acetonitrile was added. The mixture was thoroughly stirred.
   2) To 90 µL of serum, 10 µL of a standard solution containing E1, E2, and E3 each at a concentration of 10 ng/mL was added, and the mixture was stirred. Substantially the same operation as in section 1) was then carried out.
   3) On an SLE column, 230 µL of the diluted serum was loaded.
   4) Next, 600 µL of hexane/ethyl acetate (50/50, v/v) was added, and the sample was eluted. The procedure was repeated three times.
2. Dryness: a stream of nitrogen for about 20 minutes
3. Derivatization
   1) MP-DNPF was dissolved in acetonitrile so as to give a 2 mg/mL solution.
   2) To the dried sample, 20 µL of DMAP (1 mg/mL, an acetonitrile solution), 10 µL of the solution of MP-DNPF, and 20 µL of acetonitrile were added.
   3) The reaction mixture was allowed to stand at 60°C (15 minutes).
   4) Dryness: a stream of nitrogen for about 10 minutes
   5) To the residue, 100 µL of 50% (v/v) aqueous acetonitrile was added to prepare a sample for LC-MS/MS analysis.

### <LC analysis conditions>

LC analysis conditions were as follows:
Apparatus: Waters, ACQUITY UPLC I-Class
Analytical column: Osaka Soda, CAPCELL CORE C18 2.1 mm I.D. × 75 mm
Elution conditions: a flow rate of 0.3 mL/min; solvent A: 0.1% formic acid-water;
solvent B: 0.1% formic acid-acetonitrile

Detailed elution conditions were as shown in Table 7.

### [Table 7]

**Table 7. Elution conditions**

| | | | | | | | |
|---|---|---|---|---|---|---|---|
| Time (min) | 0 | 1 | 2.1 | 2.11 | 4.0 | 4.01 | 5.5 |
| B (%) | 20 | 20 | 50 | 95 | 95 | 20 | 20 |

### <MS/MS analysis conditions>

MS/MS analysis conditions were as follows:
Apparatus: Waters, Xevo TQ-XS triple-quadrupole mass spectrometer
Ionization conditions: ESI positive ion mode

SRM parameters were as shown in Table 8.

### [Table 8]

**Table 8. SRM parameters**

| | SRM (*m*/*z*) | CE |
|---|---|---|
| MP-DNP-E1 | 549.3 > 502.4 | 42 |
| MP-DNP-E1-¹³C₃ | 552.3 > 505.4 | 42 |
| MP-DNP-E2 | 551.4 > 504.2 | 42 |
| MP-DNP-E2-¹³C₃ | 554.3 > 507.2 | 42 |
| MP-DNP-E3 | 567.4 > 520.3 | 42 |
| MP-DNP-E3-¹³C₃ | 570.4 > 523.3 | 42 |

### <Analytical results>

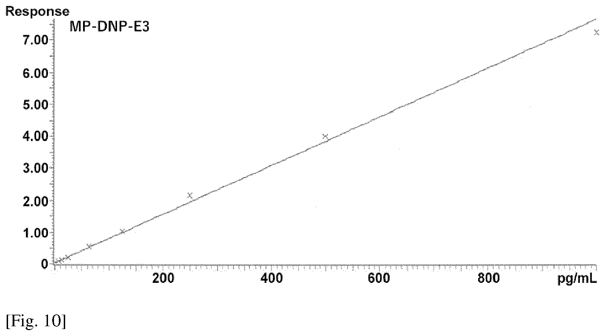

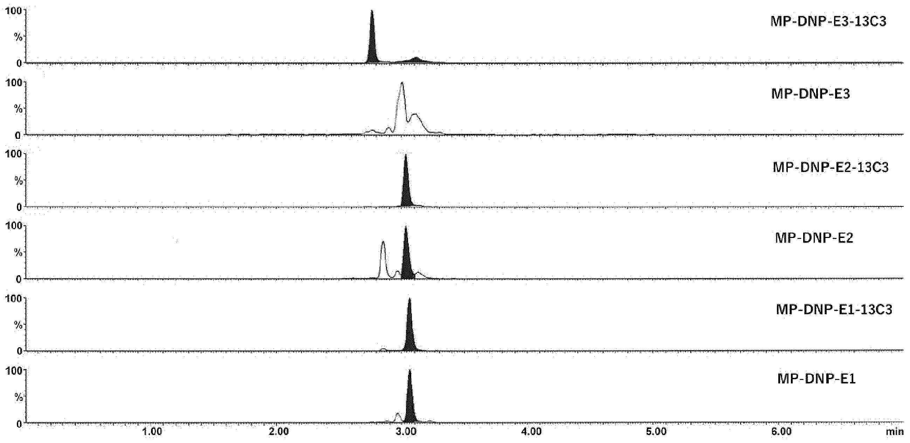

Fig. 10 shows SRM chromatograms when a pooled serum was derivatized with MP-DNPF.

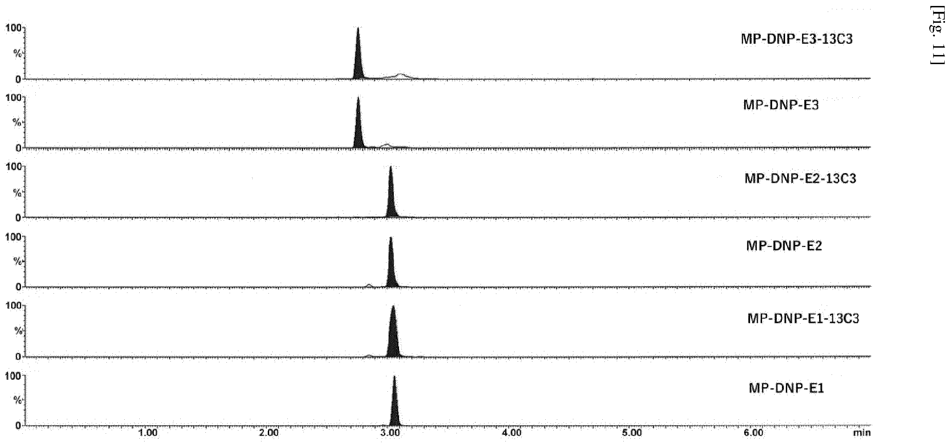

Fig. 11 shows SRM chromatograms when a serum containing standard samples were derivatized with MP-DNPF.

As shown in Fig. 10, E3 was not detected in the pooled serum (see "MP-DNP-E3" in Fig. 10). This is because the concentration of E3 in the pooled serum was extremely low. In contrast, as shown in Fig. 11, E1, E2, and E3 were simultaneously detected in the serum that had been prepared by adding E1, E2, and E3 standard samples to a pooled serum. It is supposed that derivatizing E1, E2, and E3 in a serum according to the present invention enables analysis of E1, E2, and E3 with high sensitivity.

### 6-6. Example 5

Tests were carried out to quantify E1 and E2 in a pooled serum.

### [Derivatization with MP-DNPF]

### <Sample pretreatment>

Samples were pretreated in the following procedure.
1. SLE (solid-liquid) extraction
   1) To 100 µL of serum, 100 µL of water and, as an internal standard, 20 µL of an E1-¹³C₃ and E2-¹³C₃ mixed solution (each concentration was 10 ng/mL) prepared in 50% (v/v) aqueous acetonitrile were added, and the mixture was thoroughly stirred.
   2) In 50% (v/v) aqueous acetonitrile, an E1 and E2 mixed standard sample was prepared at a concentration of 7.9 pg/mL, 15.9 pg/mL, 31.8 pg/mL, 62.5 pg/mL, 125 pg/mL, or 500 pg/mL. To 100 µL of the mixed standard sample, 100 µL of water and, as an internal standard, 20 µL of an E1-¹³C₃ and E2-¹³C₃ mixed solution (each concentration was 10 ng/mL) prepared in 50% (v/v) aqueous acetonitrile were added, and the mixture was thoroughly stirred.
   3) On an SLE column, 220 µL of the diluted serum was loaded.
   4) Next, 600 µL of hexane/ethyl acetate (75/25, v/v) was added, and the sample was eluted. The procedure was repeated twice.
2. Dryness: a stream of nitrogen for about 20 minutes
3. Derivatization
   1) MP-DNPF as a derivatization reagent was dissolved in acetonitrile so as to give a 2 mg/mL solution.
   2) To the residue, 20 µL of DMAP (1 mg/mL, an acetonitrile solution), 10 µL of the MP-DNPF solution, and 20 µL of acetonitrile were added.
   3) The reaction mixture was allowed to stand at 60°C (15 minutes).
   4) Dryness: a stream of nitrogen for about 10 minutes
   5) To the dried sample, 50 µL of 30% (v/v) aqueous acetonitrile was added to prepare a sample for LC-MS/MS analysis.

### <LC analysis conditions>

LC analysis conditions were as follows:
Apparatus: Agilent, 1290 Infinity liquid chromatography system
Analytical column: Osaka Soda, CAPCELL CORE C18 2.1 mm I.D. × 75 mm
Elution conditions: a flow rate of 0.4 mL/min; solvent A: 0.1% formic acid-water;
solvent B: 0.1% formic acid-acetonitrile

Detailed elution conditions were as shown in Table 9.

### [Table 9]

**Table 9. Elution conditions**

| | | | | | | | | |
|---|---|---|---|---|---|---|---|---|
| Time (min) | 0 | 1 | 3.1 | 4.0 | 4.01 | 5.5 | 5.51 | 6.5 |
| B (%) | 20 | 20 | 40 | 45 | 95 | 95 | 20 | 20 |

### <MS/MS analysis conditions>

MS/MS analysis conditions were as follows:
Apparatus: SCIEX, QTRAP4500
Ionization conditions: ESI positive ion mode

SRM parameters were as shown in Table 10.

### [Table 10]

**Table 10. SRM parameters**

| | SRM (*m*/*z*) | CE |
|---|---|---|
| MP-DNP-E1 | 549.3 > 502.4 | 42 |
| MP-DNP-E1-¹³C₃ | 552.3 > 505.4 | 42 |
| MP-DNP-E2 | 551.4 > 504.2 | 42 |
| MP-DNP-E2-¹³C₃ | 554.3 > 507.2 | 42 |

### <Analytical results>

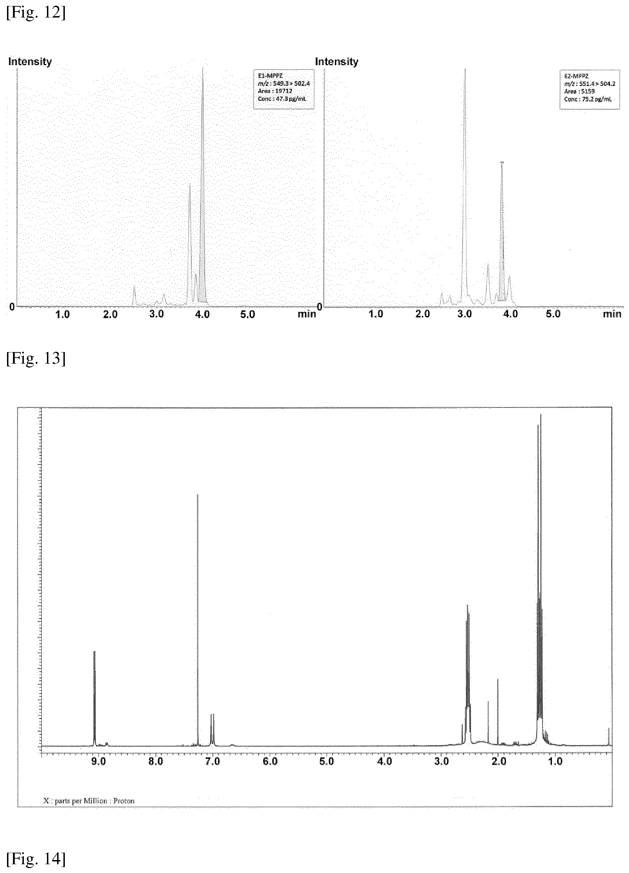

Fig. 12 shows SRM chromatograms when a pooled serum was derivatized with MP-DNPF.

Analytical results reveal that the present invention enables quantification of E1 and E2 in a pooled serum.

### 6-7. Example 6

In accordance with the following procedure, (5-fluoro-2,4-dinitrophenyl)triethylphosphonium fluoride (TEP-DNPF) was synthesized.

In 1 mL of acetonitrile, 1,5-difluoro-2,4-dinitrobenzene (100 mg, 0.49 mmol) was dissolved. The solution was stirred in an ice bath, and a solution of triethylphosphine in tetrahydrofuran (1 M 0.49 mL, 0.49 mmol) was added. Immediately after the addition, white precipitates were appeared. The reaction mixture was stirred at room temperature for 30 minutes and then was concentrated by using a rotary evaporator. The residue was purified by silica gel column chromatography (column size: 2 cm in diameter, 5 cm in length; 50% ethyl acetate-hexane, 10% methanol-chloroform) to give (5-fluoro-2,4-dinitrophenyl)triethylphosphonium fluoride (65 mg, 0.2 mmol). The structure of (5-fluoro-2,4-dinitrophenyl)triethylphosphonium fluoride is represented by Formula (1). In Formula (1), X is the group represented by Formula (5), and in Formula (5), R¹, R², and R³ are each the group represented by Formula (6) (m = 1).

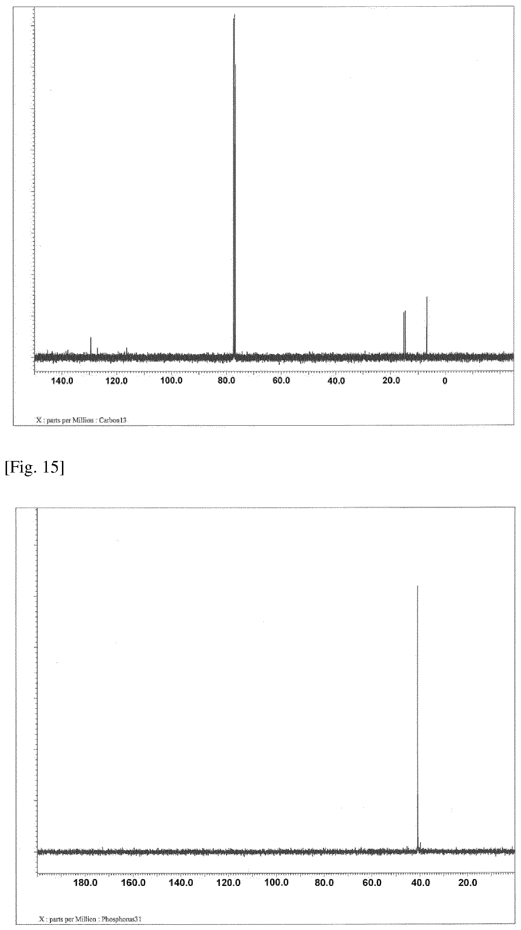

The prepared (5-fluoro-2,4-dinitrophenyl)triethylphosphonium fluoride was subjected to ¹H-NMR measurement, ¹³C- NMR measurement, and ³¹P- NMR measurement. These measurement results are as follows:
¹H-NMR (CDCl₃) δ 9.07 (d, ⁴J_{FH} = 7.2 Hz, 1H), 7.0 (d, ³J _{FH}= 17.2 Hz, 1H), 2.53 (dq, ²J_{PH}= 11.2 Hz, ³J_{HH}= 8 Hz, 6H), 1.27 (dt, ³J_{PH}= 12 Hz, ³J_{HH}= 7.6 Hz, 9H) (Fig. 13); ¹³C-NMR (CDCl₃) δ 145.3, 137.9, 129.5, 129.4, 127.0, 14.9, 6.8 (Fig. 14); ³¹P-NMR (CDCl₃) δ 41.0 (Fig. 15).

## Claims

1. An estrogen derivatization method using a quaternary cation-containing 5-fluoro-2,4-dinitrophenyl compound represented by Formula (1): 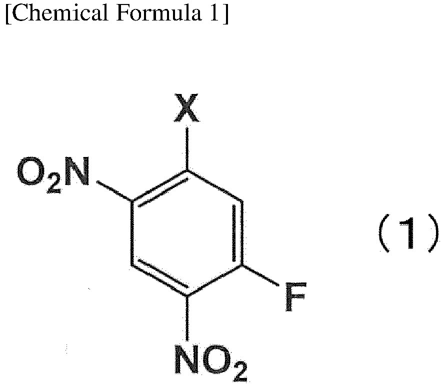 [in Formula (1), X is a quaternary cation].

2. The estrogen derivatization method according to claim 1, wherein in Formula (1), the quaternary cation is a quaternary ammonium cation.

3. The estrogen derivatization method according to claim 2, wherein the quaternary ammonium cation is a group represented by any of Formulae (2) to (4): 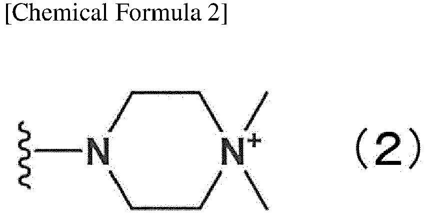 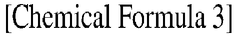 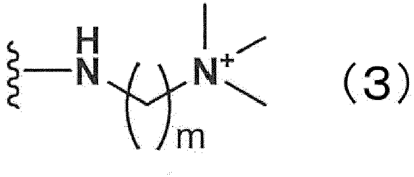 [in Formula (3), m is an integer of 1 to 8] 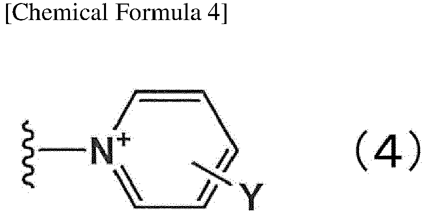 [in Formula (4), Y is a substituted or unsubstituted, linear or branched alkyl group having 1 to 20 carbon atoms].

4. The estrogen derivatization method according to claim 1, wherein in Formula (1), the quaternary cation is a quaternary phosphonium cation.

5. The estrogen derivatization method according to claim 4, wherein the quaternary phosphonium cation is a group represented by Formula (5): 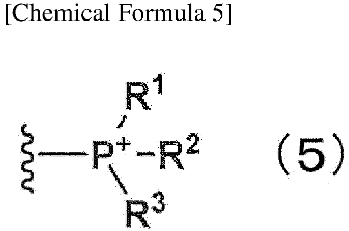 [in Formula (5), R¹, R², and R³ are each independently a group represented by Formula (6): 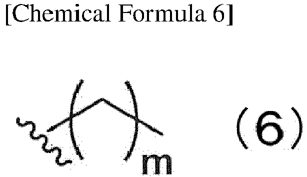 in Formula (6), m is an integer of 0 to 8].

6. The estrogen derivatization method according to claim 1, further using an organic base.

7. The estrogen derivatization method according to claim 6, wherein the organic base is a tertiary amine.

8. A mass spectrometry method comprising derivatizing estrogen by the estrogen derivatization method according to any one of claims 1 to 7.

9. A derivatization reagent for derivatizing estrogen, the derivatization reagent comprising a quaternary cation-containing 5-fluoro-2,4-dinitrophenyl compound represented by Formula (1): 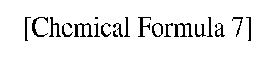 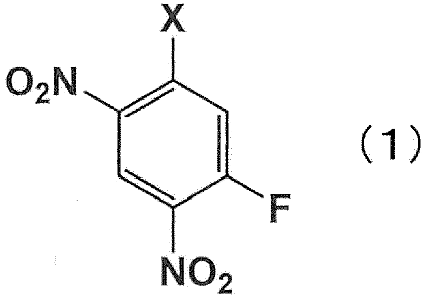 [in Formula (1), X is a quaternary cation].

10. A derivatization reagent kit for derivatizing estrogen, the derivatization reagent kit comprising the derivatization reagent according to claim 9.
